(19)

Europäisches
Patentamt
European
Patent Office
Office européen
des brevets

(11)     **EP 2 159 819 B1**

(12)               **EUROPEAN PATENT SPECIFICATION**

(45) Date of publication and mention
of the grant of the patent:
**01.08.2018 Bulletin 2018/31**

(51) Int Cl.:
*H01J 37/32* *(2006.01)*     *A61B 18/00* *(2006.01)*
*H05H 1/46* *(2006.01)*

(21) Application number: **09151534.6**

(22) Date of filing: **28.01.2009**

(54) **Plasma generator**

Plasmagenerator

Générateur de plasma

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **26.08.2008 KR 20080083364**

(43) Date of publication of application:
**03.03.2010 Bulletin 2010/09**

(73) Proprietor: **Postech Academy-Industry-
Foundation
Pohang-si, Gyeongsangbuk-do 790-784 (KR)**

(72) Inventors:
• **Choi, Jun
Gyeongsangbuk-do (KR)**

• **Iza, Felipe
Loughborough, Leicestershire LE11 3TU (GB)**
• **Koo, Jae
Gyeongsangbuk-do (KR)**

(74) Representative: **Neobard, William John
Kilburn & Strode LLP
Lacon London
84 Theobalds Road
London WC1X 8NL (GB)**

(56) References cited:
**EP-A1- 1 734 798     WO-A2-2005/098083
JP-A- 6 188 094      JP-A- 2006 202 662
US-A- 4 924 061      US-A1- 2006 042 547**

**Description**

The present invention relates to the field of plasma generation. Embodiments relate to a small-sized microwave plasma generator capable of generating plasma with low electric power, for example a portable generator..

A plasma torch is a device that generates a directed flow of plasma from its nozzle. The plasma jet can be used to melt a solid, or evaporate a solid or a liquid, or heat a gas to increase enthalpy in a closed thermodynamic system under constant and entropy.

A conventional microwave plasma generator, usually equipped with a magnetron, consumes more than 100 watts to operate. A conventional microwave plasma generator, equipped with a rectangular waveguide, is too bulky to carry. A coaxial microwave plasma torch, equipped with an antenna-shaped discharge tube may substitute for the conventional microwave plasma generator, but is also too large-sized to carry.

A conventional microwave plasma generator is currently in use which generates plasma at atmospheric pressure, using various types of electric power sources. A great deal of research has gone into developing a microwave plasma generator which generates plasma without thermal effect with low electric power by using a microwaves with a frequency of 900 MHz or 2.45 GHz.

Worldwide research has been done on application of plasma to the biomedical field. The U.S. FDA approved the use of the microwave plasma generator in medical treatment of wrinkles and speckles on the human skin. Development of a portable microwave plasma generator consuming less than 5 watts at atmospheric pressure will expand its application to cancer treatment, dental care, cosmetic treatment, disinfection, coagulation, sterilization, air purification, and so forth.

JP 06-188094 discloses a co-axial type resonator having an internal conductor within an outer casing is provided. The length of the internal conductor is set to be $(\lambda/2)n+(\lambda/4)$ microwave modulated to coaxial mode is then transmitted to the coaxial type resonator, so that plasma is generated at tip of the internal conductor.

JP 2006 202662 discloses a nearly-cylindrical metallic outer conductor having a narrowed tip and forming an outer shell part of a coaxial cavity. The outer conductor, the insulator and a metallic plasma material gas introduction tube are each formed into a tubular shape and arranged so as to be coaxial with one another. The outer conductor and the plasma material gas introduction tube are electrically connected together in the vicinity of a variable mechanism. An introduction opening of the plasma material gas introduction tube is arranged in the rearmost part of this plasma generator, and a gas outlet of the plasma material gas introduction tube is arranged on the side opposite thereto. A metallic projection member is arranged in a further front part of the gas outlet and facilitates the concentration of an electric field.

EP 1 734 798 A1 discloses a coaxial microwave plasma torch, comprising an outside conductor formed in a cylindrical shape, a cylindrical electric discharge tube fixedly inserted into an axial hole formed in the outside conductor on one end face side, and a coaxial cable having one end fitted to the other end face of the outside conductor. An antenna electrically connected to an inside conductor is fitted to the one end of the coaxial cable and extended into the electric discharge tube through a through-hole axially passed through between the other end face of the outside conductor and the bottom face of the axial hole. The outside conductor of the coaxial cable is electrically connected to the outside conductor, and a gas inlet pipeline supplying a gas into the electric discharge tube is fitted in the outside conductor.

US 4 924 061 A discloses a microwave torch comprising at least one gas supply conduit; a resonant cavity forming around the conduit a sleeve open adjacent to the outlet of the conduit and including a lateral opening; a transition coaxial structure perpendicular to the sleeve comprising, on one hand, an outer tube connected to the lateral opening of the sleeve and, on the other hand, an inner element having one end in contact with the conduit and an opposite end which is in contact with the outer surface of a wave guide and carries a transition member disposed in the wave guide; said wave guide supplying microwave energy and having a rectangular section and perpendicular to the coaxial structure being provided with an opening where the outer tube of the coaxial structure is connected; shielding gas supply means in the wave guide and/or the coaxial structure and/or the sleeve; optionally tuning means; and optionally plasma igniting means.

US 2006/052547 A1 discloses a portable microwave plasma discharge unit for receiving microwaves and a gas flow via a supply line. The portable microwave plasma discharge unit generates plasma from the gas flow and the received microwaves. The portable microwave plasma discharge unit includes a gas flow tube made of a conducting and/or dielectric material and a rod-shaped conductor that is axially disposed in the gas flow tube. The rod-shaped conductor has an end configured to contact a microwave supply conductor of the supply line to receive microwaves and a tapered tip positioned adjacent the outlet portion of the gas flow tube. The tapered tip is configured to focus the microwaves received from the microwave supply conductor to generate plasma from the gas flow.

WO 2005/098083 A2 discloses a miniature microwave plasma torch apparatus for a variety of applications where rapid heating of a small amount of material is needed. The miniature microwave plasma torch apparatus operates near or at atmospheric pressure for use in materials processing. The apparatus provides a wide range of flow rates so that discharge properties vary from diffusional flow of radicals for gentle surface processing to high velocity, approaching supersonic, torch discharges for cutting and welding applications.

Embodiments aim to provide a small-sized portable microwave plasma generator capable of generating plasma at atmospheric pressure with low electric power.

There is provided a portable microwave plasma generator capable of generating plasma with low electric power. Including a coaxial cable, an outer conductor, a connection conductor; and a connection member. The coaxial cable includes a first inner conductor and a dielectric material encircling the first inner conductor. The outer conductor encircles the coaxial cable and includes

the connection conductor and the connection member. The connection conductor includes at least one gas inlet tube and electrically connects between the first inner conductor and the outer conductor at one end of the coaxial cable. The connection member includes a second inner conductor passing through the outer conductor and then connecting to the first inner conductor at a feeding point, wherein the feeding point is positioned such that a required impedance matching is provided.

In an embodiment, the microwave plasma generator is less than 10 cm in length and generates plasma at atmospheric pressure with low electric power. In an embodiment, the microwave plasma generator is capable of matching its impedance with impedance of a microwave oscillator supplying a microwave without having to use a separate impedance matching device.

An embodiment of the invention will be described with reference to the accompanying drawings, in which:

FIG.1 is a cross-sectional view of a portable microwave plasma generator capable of generating plasma with low electric power;

FIG.2 is a front view of the A direction end of the portable microwave plasma generator capable of generating plasma with low electric power, as shown in FIG.1;

FIG.3 is a front view of the B direction end of the portable microwave plasma generator capable of generating plasma with low electric power, as shown in FIG.1;

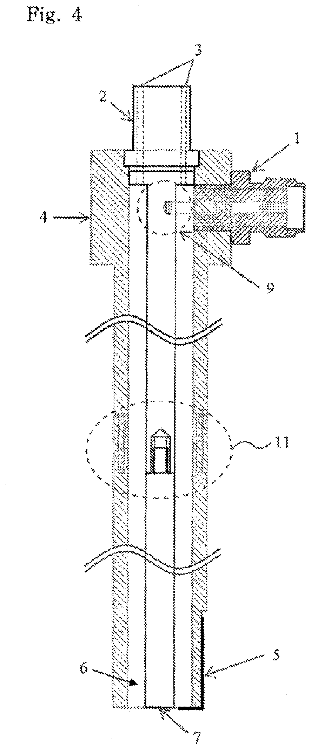

FIG.4 is a view of the portable microwave plasma generator capable of generating plasma with low electric power, having the coaxial cable whose the length is adjustable;

FIG.5 is a schematic view of the portable microwave plasma generator capable of generating plasma with low electric power;

FIG.6 is a picture of an actual product embodying the portable microwave plasma generator capable of generating plasma with low electric power;

FIG.7 is a front view of the right end F of the portable microwave plasma generator capable of generating plasma with low electric power, as shown in FIG.6; and

FIG.8 is a front view of the left end R of the portable microwave plasma generator capable of generating plasma with low electric power, as shown in FIG.6.

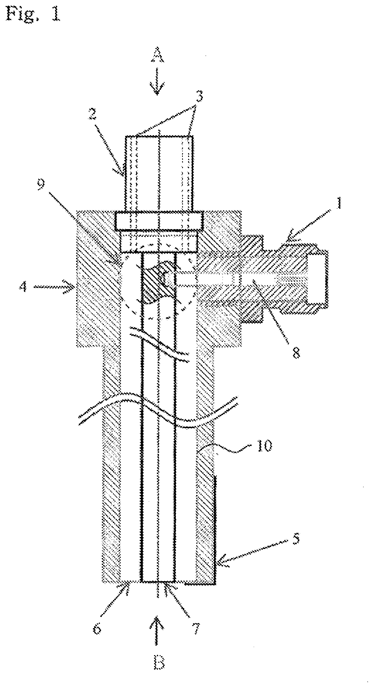

Referring to FIG.1, a portable microwave plasma generator 100 includes a coaxial cable 10, an outer conductor 4, a connection conductor 2, a connection member 1, and a discharge tip 5,

The coaxial cable 10 includes a first inner conductor 7 and a dielectric material 6 encircling the first inner conductor 7. The outer conductor encircles the coaxial cable 10. The connection conductor 2 includes at least one gas inlet tube 3. The connection conductor 2 electrically connects between the first inner conductor 7 and the outer conductor 4 at one end of the coaxial cable 10. The connection member 1 includes a second inner conductor 8 passing through the outer conductor 4 and then electrically connects to the first inner conductor 7 at a feeding point 9. The connection member 1 is conductive. An insulating material may be provided between the second inner conductor 8 and the connection member 1. The discharge tip 5, provided on the other end of the coaxial cable 10, serves to minimize electric power consumption when generating plasma on the coaxial cable 10.

The portable microwave plasma generator 100 generates plasma using resonance energy of a microwave with a frequency of 900 MHz or 2.45 GHz which is applied through the second inner conductor 8. The connection member 1, the connection conductor 2, the outer conductor 4, the first inner conductor 7, and the second inner conductor 8 electrically connects to each other and function as a resonator oscillating the microwave. The microwave converts into a TEM (Transverse Electro-Magnetic) wave in the coaxial cable 10, The TEM wave is a transverse wave, carrying electromagnetic energy, which has not an electric field component and a magnetic field component in the propagation direction, but has the electric field component and the magnetic field component in the direction perpendicular (transverse) to the propagation direction.

Air is used as the dielectric material 6 between an outer jacket of the coaxial cable 10 and the first inner conductor 7. That is, the dielectric 6 is an empty space into which an inert gas is introduced to be ionized. The introduced gas becomes in a state of plasma by resonance energy of the microwave resonating and amplified on the conductors 2, 4, 7, and 8 to which the microwave is applied, and discharges toward the bottom of the portable microwave plasma generator, i.e., in the direction B. Other material such as a fluorocarbon solid (for example PTFE) may be used as the dielectric material 6.

A length of the coaxial cable 10 may be 1/4 (one-third) or 3/4 (three-quarters) of a wavelength of the microwave which is suitable for the microwave to produce resonance. When the length of the coaxial cable 10 is set to this length, the electric field strength becomes maximized at the end of the resonator which is in the direction B, thereby generating plasma more easily.

The relation between a speed of light, a frequency and a length of the microwave is expressed as the following equation. 1.

$$[ \text{ EQUATION 1}]$$

$$\lambda = \frac{C}{f \cdot \sqrt{\varepsilon r}}$$

where A is a wavelength of the microwave, C is a speed of light, f is a frequency of the microwave, and $\varepsilon r$ is a relative dielectric constant. The relative dielectric constant is 1 (one) when the dielectric material is an air and is 2.1 when the dielectric material is PTFE.

For example, the frequency of the microwave is 900MHz, the length $\lambda$ of the microwave is expressed as the following equation 2.

$$[ \text{ EQUATION 2}]$$

$$\lambda = \frac{3 \cdot 10^8}{900 \cdot 10^8} = 0.333[m] \approx 33.3[cm]$$

According to the equation 2, 1/4 (one-quarter) of the microwave is approximately 8.33 cm. This makes it possible to manufacture the portable microwave plasma generator which is around 10cm in length.

An input impedance of the coaxial cable 10 seen from the feeding point 9 where the first inner conductor 7 of the coaxial 10 and the second inner conductor 8 of the connection member 1 come in contact with each other varies depending upon a position of the feeding point 9. By adjusting the position of the feeding point 9, the impedance matching can easily be made between a microwave oscillator (not shown) applying the microwave to the coaxial cable 10 through the second inner conductor 8 and the microwave plasma generator or between an amplifier amplifying the microwave outputting from the microwave oscillator and the microwave plasma generator. This is later described in more detail.

Generation of plasma at atmospheric pressure requires high electric field strength of $10^6$ V/m (Volts/meter) or more. The discharge tip 5 is used to locally increase the electric field strength. The discharge tip 5 does not need to be used, after discharge occurs and plasma is generated. The discharge tip 5, when not in use, may be removed from the microwave plasma generator. Experimental findings show that an embodiment of the portable microwave plasma generator can generate plasma with about 1 watt or so.

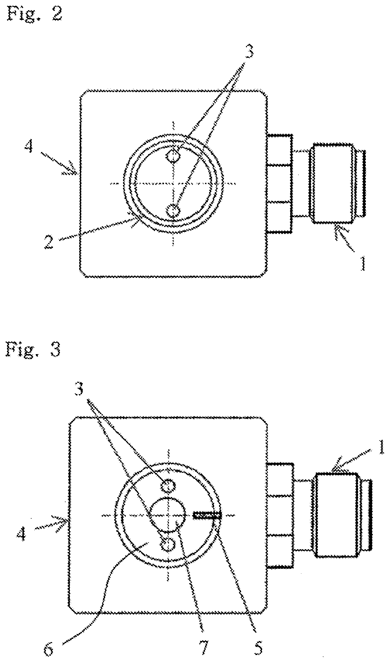

As shown in FIG2, the connection conductor 2 is inserted into the middle of the outer conductor 4. And the two gas inlet tubes 3, through which the inert gas is introduced into the dielectric material 6, are provided to the connection conductor 2. The single inlet tube 3 may be provided to the connection conductor 2. The inert gas includes helium and argon.

As shown in FIG3, the outer conductor 4 encircles the coaxial cable 10 which has the first inner conductor 7 at the center, and the dielectric material 6 is provided between the outer jacket of the coaxial cable 10 and the first inner conductor 7. The discharge tip 5 is positioned on the end of the coaxial cable 10, which is in the direction B. The discharge tip 5 discharges plasma outputting in the direction B.

As shown in FGI.4, a coaxial cable extension tab 11, oval-shaped part (marked dotted line) in the middle of the portable microwave plasma generator 400 serves to adjust the length of the outer conductor 4 of the coaxial cable and the length of the first inner conductor 7 of the coaxial cable. For example, the outer conductor 4 of the coaxial cable 10 and the first inner conductor 7 of the coaxial cable 10 may be expandable or contractable in a slidable manner or in an attachable and detachable manner to adjust the length of the coaxial cable 10. Adjustment of the length of the coaxial cable 10 with the coaxial cable extension tab 11 makes it possible not only to effectively produce resonance, but also to make the impedance matching which prevents the microwave from being reflected. The lower part of the coaxial cable 10 and the upper part of the coaxial part including the extension tab 11 may attach to or detach from each other. This makes it possible to replace of the coaxial cable 10 including the extension tab 11, when the end of the resonator is eroded away by plasma, thereby extending the life of the microwave plasma generator.

An embodiment of the portable microwave plasma generator capable of generating plasma with low electric power can adjust its impedance by adjusting the position of the connection member 1. This removes the need for installing the impedance match device to make the impedance matching between the microwave oscillator (not shown) applying the microwave to the coaxial cable 10 through the second inner conductor 8 and the microwave plasma generator or between an amplifier amplifying the microwave outputting from the microwave oscillator and the microwave plasma generator.

Operation of the portable microwave plasma generator and relation between it and its peripheral devices are now described.

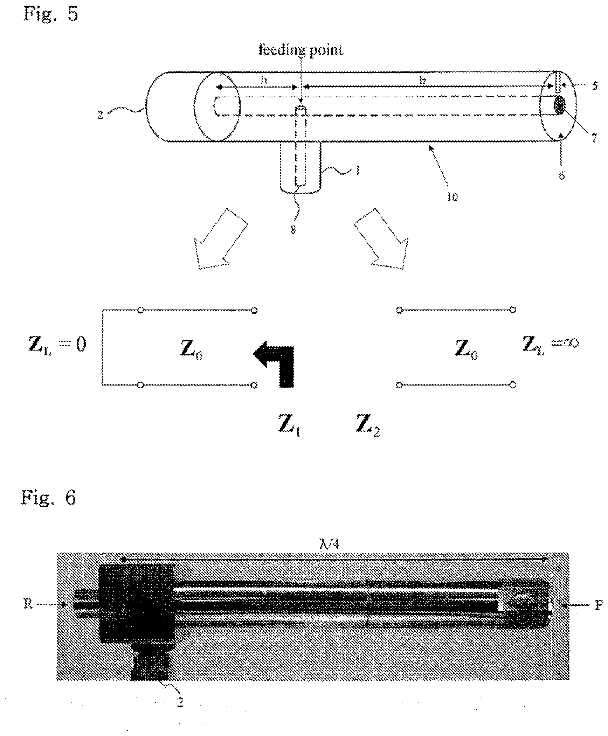

Referring to FIG5, the input impedance $Z_{IN}$ of the coaxial cable 10 seen from the feeding point 9 where the first inner conductor 7 of the coaxial cable 10 and the second inner conductor 8 of the connection member 1 come in contact with each other is expressed as the following equation 3.

[ EQUATION 3]

$$Z_{IN} = Z_1 \| Z_2 = Z_0 \left[ \frac{1}{\tanh(jkl_1)} + \tanh(jkl_2) \right]^{-1}$$

$$\approx Z_0 \left[ \left( \frac{\alpha l_1}{\sin^2(\beta l_1)} + \frac{\alpha l_2}{\cos^2(\beta l_2)} \right) + j(-\cot(\beta l_1) + \tan(\beta l_2)) \right]$$

Where the first impedance $Z_1$ is an impedance of the coaxial cable 10 to the left of the feeding point 9, a second impedance $Z_2$ is an impedance of the coaxial cable to the right of the feeding point 9, $Z_0$ is a characteristic impedance of the coaxial cable 10, j is a complex number $\sqrt{-1}$, $l_1$ is a distance between the feeding point 9 and the left end of the coaxial cable 10, $l_2$ is a distance between the feeding point 9 and the right end of the coaxial cable 10, k is a complex propagation constant or a wave number which is a reciprocal number of wavelength, $\alpha$ is an attenuation constant of the coaxial cable 10, and $\beta$ is a phase constant of the coaxial cable 10. The equation 3 is written on the assumption that when $(\alpha l)=1$(one), $\tan(\alpha l) \approx (\alpha l)$.

The left end of the coaxial cable 10 connects to the connection conductor 2, so a load impedance $Z_L$ of the first impedance $Z_1$ is 0 (zero). The right end of the coaxial cable 10 opens, so the load impedance is $\infty$ (infinite). The attenuation constant $\alpha$ of the coaxial cable 10, and the phase constant $\beta$ of the coaxial cable 10 are constants which are automatically determined by the electric characteristics of the coaxial cable 10. So, the input impedance $Z_{IN}$ i.e., the input impedance $Z_{IN}$ of the resonator is determined by $l_1$ and $l_2$.

When the length of the coaxial cable 10 is less than 1/4 (one-quarter) of the wavelength of the microwave, adjustment of $l_1$ means adjustment of inductance of the resonator, and adjustment of $l_2$ means adjustment of capacitance of the resonator. So, the resonator corresponds to what consists of the resistor, the inductor, and the capacitor which connect in parallel to each other. Thus, the position of the feeding point 9 is determined by adjusting any one of $l_1$ and $l_2$. When the input impedance $Z_{IN}$ is set to 50Ω(Ohm), the separate impedance matching device does not need to be installed.

This is about the coaxial cable 10 of which the length is 1/4 (one-quarter) or 3/4 (three-quarters) of the wavelength of the microwave. This may be applied to the coaxial cable 10 of which the length is any one of multiples of 1/4 (one-quarter) and multiples of 3/4 (three-quarters) of the wavelength of the microwave.

As shown in FIG.6, the length of the coaxial cable 10 in the portable microwave plasma generator is 1/4 (one-quarter) of wavelength of the microwave. A SMA (Sub Miniature version A) connector is used as the connection member 1 .

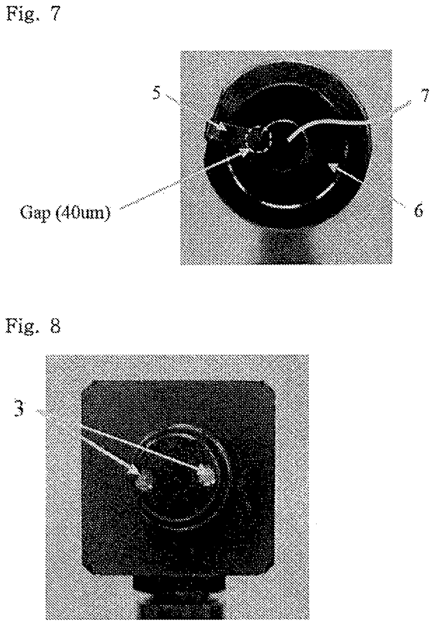

FIG.7 is a front view of the right end F of the portable microwave plasma generator capable of generating plasma with low electric power, as shown in FIG.6.

As shown in FIG.7, there is a given gap between the discharge tip 5 and the first inner conductor 7 at the right end of the portable microwave plasma generator. If the portable microwave plasma generator makes resonance using the microwave with a frequency of 900 MHz and the length of the coaxial cable 10 is 1/4 (one-quarter) of the wavelength of the microwave, then the gap is 40 $\mu$m If the portable microwave plasma generator makes resonance using the microwave with a frequency of 2.45 GHz and the length of the coaxial cable 10 is 3/4 (three-quarters) of the wavelength of the microwave, then the gap is 100 $\mu$m.

As shown in FIG.8, the inert gas is introduced into the gas inlet tube 3.

As above described, the coaxial cable 100 designed for the specific purpose is employed in the portable microwave plasma generator according to the present invention. However, a general type of the semi rigid coaxial cable in wide use may be instead employed. The voltage and the electromagnetic field become at a maximum at the end of the resonator, i.e. at the end of the portable microwave plasma generator. As a result, the portable microwave plasma generator effectively generates plasma, using electric power at a minimum, compared with the conventional microwave plasma generator. Furthermore, the length of the portable microwave plasma generator is made to be 10 cm or so that corresponds to one-quarter of the wavelength of the microwave. This makes portable the portable microwave plasma generator. The portable microwave plasma generator does not require the installation of separate impedance matching device, if the impedance of the resonator is adjusted to 50 Ω, the output impedance of the microwave oscillator.

**Claims**

1.  A microwave plasma generator (100) comprising:
    a coaxial cable (10) comprising:

        a first inner conductor (7); and
        a dielectric material (6) encircling the first inner conductor;
        an outer conductor (4) encircling the coaxial cable; and **characterised by** including:

            a connection conductor (2), comprising at least one gas inlet tube (3), electrically connecting between the first inner conductor and the outer conductor at one end of the coaxial cable; and
            a connection member (1) comprising a second inner conductor (8) passing through the outer conductor and then connecting to the first inner conductor at a feeding point (9),
            wherein the feeding point is positioned such that a required impedance matching is provided.

2.  A plasma generator according to claim 1, wherein the dielectric material is air, an empty space into which to introduce an inert gas, and a microwave is applied through the second inner conductor.

3.  A microwave plasma generator according to claim 2, wherein the inert gas is selected from a group consisting of helium and argon.

4.  A microwave plasma generator according to claim 2, wherein the microwave has a frequency of 900MHz or 2.45GHz.

5.  A microwave plasma generator, according to claim 2, wherein a length of the coaxial cable (10) is 1/4 (one-quarter), or 3/4 (three-quarters) of the wavelength of the microwave.

6.  A microwave plasma generator, according to claim 2, wherein a length of the coaxial cable (10) is any one of multiples of 1/4 (one-quarter) and multiples of 3/4 (three-quarters) of the wavelength of the microwave.

7.  A microwave plasma generator according to claim 1, wherein the coaxial cable (10) comprises at least two parts which are attachable to and detachable from each other or are slidable into each other in a manner that adjust the length of the coaxial cable.

8.  A microwave plasma generator according to claim 4, further comprising a discharge tip (5), provided on the other end of the coaxial cable, for discharging plasma in the coaxial cable.

9.  A microwave plasma generator according to claim 8,
    wherein a gap between the discharge tip (5) and the first inner conductor is $40\mu$m when the microwave has a frequency of 900MHz.

10. A microwave plasma generator according to claim 8, wherein a gap between the discharge tip (5) and the first inner conductor is $100\mu$m when the microwave has a frequency of 2.45GHz.

11. A microwave plasma generator according to any preceding claim, wherein the generator is portable.

**Patentansprüche**

1.  Mikrowellenplasmagenerator (100), umfassend:

ein Koaxialkabel (10), umfassend:

einen ersten Innenleiter (7); und
ein dielektrisches Material (6), das den ersten Innenleiter umschließt;
einen Außenleiter (4), der das Koaxialkabel umschließt; und **dadurch gekennzeichnet, dass** er einschließt:

einen Verbindungsleiter (2), der mindestens ein Gaseinlassrohr (3) umfasst, zwischen dem ersten Innenleiter und dem Außenleiter an einem Ende des Koaxialkabels elektrisch verbindend; und
ein Verbindungselement (1), einen zweiten Innenleiter (8) umfassend, der durch den Außenleiter verläuft und sich dann an einem Einspeisepunkt (9) mit dem ersten Innenleiter verbindet,
wobei der Einspeisepunkt so positioniert ist, dass ein erforderlicher Impedanzabgleich bereitgestellt wird.

2. Plasmagenerator nach Anspruch 1, wobei das dielektrische Material Luft ist, ein leerer Raum, in den ein Inertgas einzuführen ist, und über den zweiten Innenleiter eine Mikrowelle angelegt wird.

3. Mikrowellenplasmagenerator nach Anspruch 2, wobei das Inertgas aus einer Gruppe ausgewählt wird, die aus Helium und Argon besteht.

4. Mikrowellenplasmagenerator nach Anspruch 2, wobei die Mikrowelle eine Frequenz von 900 MHz oder 2,45 GHz aufweist.

5. Mikrowellenplasmagenerator nach Anspruch 2, wobei eine Länge des Koaxialkabels (10) 1/4 (ein Viertel) oder 3/4 (drei Viertel) der Wellenlänge der Mikrowelle beträgt.

6. Mikrowellenplasmagenerator nach Anspruch 2, wobei eine Länge des Koaxialkabels (10) ein beliebiges von Vielfachen von 1/4 (ein Viertel) und Vielfachen von 3/4 (drei Viertel) der Wellenlänge der Mikrowelle beträgt.

7. Mikrowellenplasmagenerator nach Anspruch 1, wobei das Koaxialkabel (10) mindestens zwei Teile umfasst, die aneinander befestigbar und voneinander trennbar sind oder auf eine Weise ineinander schiebbar sind, die die Länge des Koaxialkabels einstellt.

8. Mikrowellenplasmagenerator nach Anspruch 4, ferner eine Entladespitze (5) umfassend, die an dem anderen Ende des Koaxialkabels bereitgestellt ist, um Plasma im Koaxialkabel zu entladen.

9. Mikrowellenplasmagenerator nach Anspruch 8, wobei ein Spalt zwischen der Entladespitze (5) und dem ersten Innenleiter 40 $\mu$m beträgt, wenn die Mikrowelle eine Frequenz von 900 MHz hat.

10. Mikrowellenplasmagenerator nach Anspruch 8, wobei ein Spalt zwischen der Entladespitze (5) und dem ersten Innenleiter 100 $\mu$m beträgt, wenn die Mikrowelle eine Frequenz von 2,45 GHz hat.

11. Mikrowellenplasmagenerator nach einem beliebigen vorhergehenden Anspruch, wobei der Generator tragbar ist.


## Revendications

1. Générateur de plasma à micro-ondes (100) comprenant :
un câble coaxial (10) comprenant :

un premier conducteur interne (7) ; et
un matériau diélectrique (6) encerclant le premier conducteur interne ;
un conducteur externe (4) encerclant le câble coaxial ; et **caractérisé en ce qu'**il comprend :

un conducteur de connexion (2) comprenant au moins un tube d'entrée de gaz (3), réalisant la connexion électrique entre le premier conducteur interne et le conducteur externe au niveau d'une extrémité du câble coaxial ; et
un élément de connexion (1) comprenant un second conducteur interne (8) passant à travers le conducteur externe, puis se connectant au premier conducteur interne au niveau d'un point d'alimentation (9),
le point d'alimentation étant positionné de telle sorte qu'une adaptation d'impédance requise est fournie.

**2.** Générateur de plasma selon la revendication 1, le matériau diélectrique étant de l'air, un espace vide dans lequel est introduit un gaz inerte, et une micro-onde étant appliquée à travers le second conducteur interne.

**3.** Générateur de plasma à micro-ondes selon la revendication 2, le gaz inerte étant choisi dans un groupe constitué d'hélium et d'argon.

**4.** Générateur de plasma à micro-ondes selon la revendication 2, la micro-onde ayant une fréquence de 900 MHz ou 2,45 GHz.

**5.** Générateur de plasma à micro-ondes, selon la revendication 2, une longueur du câble coaxial (10) étant 1/4 (un quart), ou 3/4 (trois quarts) de la longueur d'onde de la micro-onde.

**6.** Générateur de plasma à micro-ondes, selon la revendication 2, une longueur du câble coaxial (10) étant l'un quelconque des multiples de 1/4 (un quart) et des multiples de 3/4 (trois quarts) de la longueur d'onde de la micro-onde.

**7.** Générateur de plasma micro-ondes selon la revendication 1, le câble coaxial (10) comprenant au moins deux parties qui sont attachables l'une à l'autre et détachables l'une de l'autre ou peuvent coulisser l'une dans l'autre d'une manière qui ajuste la longueur du câble coaxial.

**8.** Générateur de plasma à micro-ondes selon la revendication 4, comprenant en outre une pointe de décharge (5), disposée sur l'autre extrémité du câble coaxial, pour décharger le plasma dans le câble coaxial.

**9.** Générateur de plasma à micro-ondes selon la revendication 8, un espace entre la pointe de décharge (5) et le premier conducteur interne étant de 40 $\mu$m lorsque la micro-onde a une fréquence de 900 MHz.

**10.** Générateur de plasma à micro-ondes selon la revendication 8, un espace entre la pointe de décharge (5) et le premier conducteur interne étant de 100 $\mu$m lorsque la micro-onde a une fréquence de 2,45 GHz.

**11.** Générateur de plasma à micro-ondes selon l'une quelconque des revendications précédentes, le générateur étant portable.

Fig. 1

Fig. 2

Fig. 3

Fig. 4

Fig. 5

Fig. 6

Fig. 7

5     7

Gap (40um)     6

Fig. 8

3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6188094 A
- JP 2006202662 A
- EP 1734798 A1
- US 4924061 A
- US 2006052547 A1
- WO 2005098083 A2